# FASCICULE DE BREVET EUROPEEN

(11) **EP 1 099 427 B1**
(45) Date de publication et mention de la délivrance du brevet: **19.01.2005**
(21) Numéro de dépôt: 00403091.2
(22) Date de dépôt: 08.11.2000
(51) Int. Cl.: A61F 2/36, A61B 17/72

(54) **Tige fémorale de reconstruction**
Wiederherstellungs-Femurschaft
Reconstruction femoral shaft

(30) Priorité: 09.11.1999 FR 9914045
(43) Date de publication de la demande: 16.05.2001
(73) Titulaire: Amplitude, 01700 Neyron (FR)
(72) Inventeur:
(74) Mandataire: Robert, Jean-Pierre

(56) Documents cités:
- EP-A- 0 587 955
- EP-A- 0 720 839
- EP-A- 0 729 732
- FR-A- 2 606 273
- FR-A- 2 705 558
- FR-A- 2 730 628
- US-A- 4 813 963
- US-A- 5 002 581
- US-A- 5 725 592

## Description

La présente invention concerne les prothèses de hanche et plus précisément les tiges fémorales de reconstruction.

On appelle tige de reconstruction la partie de prothèse qui est implantée dans le fémur après un descellement d'une tige précédente ayant entraîné ( ou ayant été causé par) une destruction plus ou moins importante de l'os au niveau de la partie métaphysaire du fémur.

Une tige de ce type est de construction modulaire. Il faut en effet, pour trouver un encrage solide de la prothèse, prévoir la mise en place d'un clou enfoncé profondément dans le canal médullaire du fémur, clou qui est ensuite immobilisé au moyen de broches transversales traversant la paroi osseuse et le clou pré-percé transversalement.

Au sommet de ce clou il est prévu d'une part un orifice taraudé longitudinal et, autour de cet orifice, une extrémité conique conçue pour recevoir une pièce métaphysaire elle-même coiffée par une pièce terminale portant le col de la prothèse (pièce épiphysaire). Ces deux pièces emboîtées l'une dans l'autre sont fixées au clou par une vis traversante qui vient en prise dans l'orifice taraudé, comme décrit dans le document US-A-5,725,592.

On comprend que plus la partie supérieure du fémur est détériorée, plus il convient de s'éloigner de l'articulation pour trouver une partie saine osseuse où implanter la prothèse. L'axe médullaire de cette partie saine constitue l'axe d'orientation générale du clou. Il n'est donc pas rare, compte-tenu de la non-linéarité de l'os naturel, que cet ancrage éloigné conduise à un déport de la pièce métaphysaire de la prothèse qui ne coïncide plus avec la métaphyse naturelle. Le chirurgien est alors contraint d'utiliser une pièce métaphysaire plus petite que celle qui aurait normalement pu être accueillie par le fémur. Cette pièce est alors éloignée des parties osseuses subsistant et l'ancrage n'est pas de très bonne qualité.

Or il est souhaitable de profiter au maximum de la paroi osseuse qui reste présente en partie supérieure du fémur. En effet, plus la transmission de la charge sera répartie entre l'insert prothétique et l'os naturel, meilleure sera la tenue de la prothèse car les points de concentration de contrainte seront diminués.

Par la présente invention on se propose de profiter la structure modulaire d'une tige de reconstruction pour offrir au chirurgien le maximum de possibilités pour s'approcher au mieux de la géométrie et de la cinématique naturelle de l'articulation à restaurer.

A cet effet l'invention a donc pour objet une tige de reconstruction pour prothèse de hanche comportant un clou longitudinal destiné à une implantation diaphysaire avec au moins un orifice transversal, une partie métaphysaire adaptable à l'extrémité supérieure du clou et une partie de col surmontant la partie métaphysaire, l'ensemble étant assemblé par l'intermédiaire d'une vis traversante commune. L'une des caractéristiques de l'invention est que la partie métaphysaire présente une asymétrie par rapport à son plan frontal médian passant par l'axe de l'orifice de la vis traversante.

Le chirurgien disposera donc pour une taille déterminée d'une partie métaphysaire symétrique, d'une partie métaphysaire renflée du côté antérieur du plan frontal et d'une partie métaphysaire renflée du côté postérieur du plan frontal afin de pouvoir correspondre au mieux avec la géométrie naturelle du fémur à reconstruire.

Afin de s'approcher au plus près de la position de l'articulation naturelle malgré une orientation non favorable du clou de la prothèse, la partie métaphysaire comporte une surface plane supérieure et un pion de centrage de cette dernière autour de l'orifice de la vis traversante, en saillie de cette surface plane et pourvu au moins en partie supérieure d'une pluralité de crans d'indexation angulaire de la partie épiphysaire autour de ce pion.

La largeur de la surface plane de la partie métaphysaire du côté de l'intrados de la prothèse c'est-à-dire du côté concave est plus importante que la largeur correspondante de la surface plane de la partie épiphysaire. Ceci permet l'ajustement angulaire des deux parties l'une par rapport à l'autre sans que la partie épiphysaire déborde latéralement de la partie métaphysaire.

En revanche il peut être intéressant que la longueur de la surface de la partie épiphysaire prise depuis l'axe de l'orifice de la vis traversante soit légèrement plus importante que cette même longueur de la surface supérieure de la partie métaphysaire afin qu'en assemblant la prothèse, la partie épiphysaire débordant de la partie métaphysaire assure une compression de l'os à l'intrados de la prothèse ce qui peut être favorable à un bon ancrage de celle-ci. La partie épiphysaire pourra alors présenter une collerette sous le col à l'effet d'augmenter sa surface inférieure pour déborder de la partie métaphysaire.

Enfin le clou possède à son extrémité supérieure un moyen d'indexation angulaire du matériel ancillaire de perçage de l'os par rapport à l'axe d'au moins l'orifice transversal susdit. Ce moyen d'indexation peut être constitué tout simplement par un fraisage transversal d'extrémité et qui permet à une lame appartenant au matériel ancillaire d'être orientée autour de l'axe du clou.

D'autres caractéristiques et avantages ressortiront de la description d'un mode de réalisation de l'invention donnée ci-après à titre d'exemple non-limitatif.

Il sera fait référence aux dessins annexés parmi lesquels :

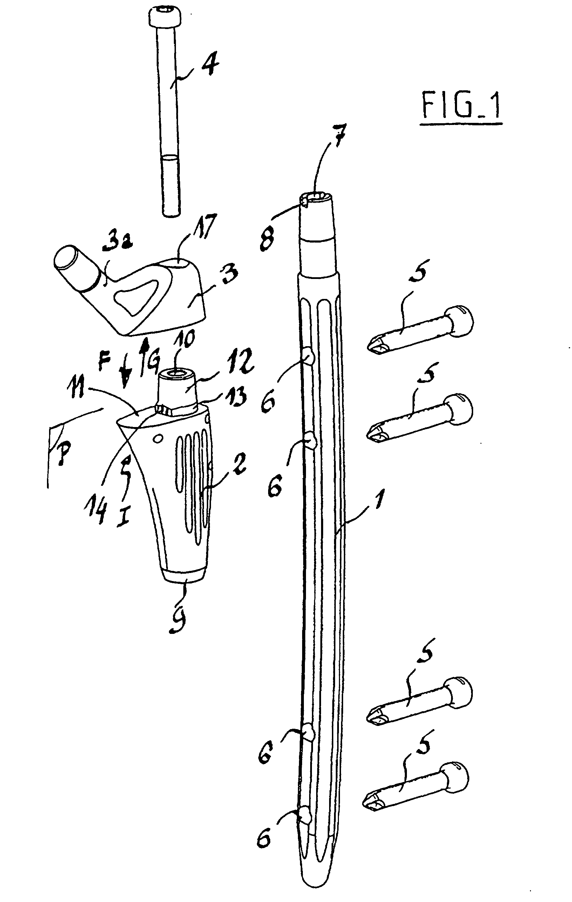

- la figure 1 est une vue éclatée d'une tige de reconstruction selon l'invention,

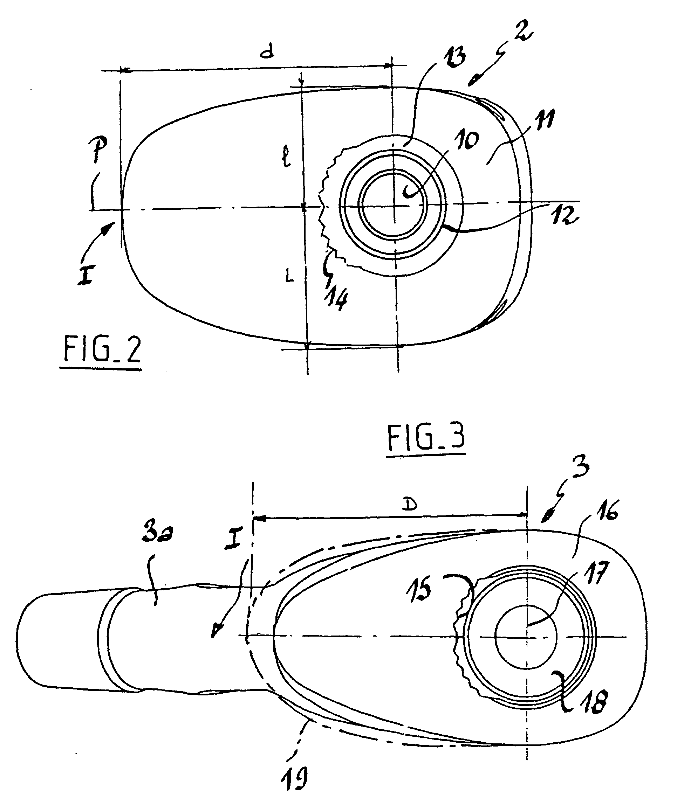

- la figure 2 est une vue suivant F de la figure 1,
- la figure 3 est une vue suivant G de la figure 1.

La prothèse de reconstruction selon l'invention comporte classiquement un clou 1, une partie métaphysaire 2, une partie épiphysaire 3 avec un col 3a, une vis d'assemblage commune 4, et quatre broches transversales 5 de fixation du clou dans la partie diaphysaire du fémur à reconstruire.

Le clou est percé d'orifices transversaux 6 destinés à accueillir les broches 5 et d'un orifice taraudé axial 7 destiné à accueillir la vis 4. Une fente 8 pourvue à la partie d'extrémité supérieure du clou 1 constitue un moyen d'indexation pour le matériel ancillaire destiné à mettre en place les broches 5 dans l'axe des orifices 6 alors que le clou est implanté dans le fémur.

La partie métaphysaire 2 selon l'invention vient coiffer l'extrémité supérieure du clou 1 au moyen d'un orifice conique qu'elle possède en sa partie inférieure 9 qui coopère avec un cône correspondant ménagé comme surface extérieure autour de l'orifice taraudé 7. L'orifice conique de la partie métaphysaire 2 se prolonge par un orifice traversant 10 pour le passage de la vis 4.

A son extrémité la plus large, la partie métaphysaire 2 est limitée par une surface plane 11 de laquelle fait saillie un pion 12, de préférence conique, de centrage de la partie épiphysaire 3. A sa base, le pion 12 comporte une collerette 13 équipée au moins partiellement d'une série de crans 14 pour coopérer avec un relief intérieur complémentaire 15 prévu dans la partie épiphysaire 3. On remarquera que la partie métaphysaire n'est pas symétrique par rapport au plan P dit plan frontal de celle-ci et que cette asymétrie se traduit par une largeur l au-dessus de ce plan inférieure à une largeur L en dessous de celui-ci.

En ce qui concerne la partie épiphysaire 3, celle-ci possède une surface plane inférieure 16 dans laquelle débouche un orifice traversant 17 pour le passage de la vis 4, cet orifice possédant un chambrage inférieur 18 conique apte à coopérer avec le pion 12, lequel chambrage est pourvu des reliefs 15 complémentaires des crans 14.

On remarquera que la largeur de la surface 16 de la partie épiphysaire 3 est globalement inférieure à la largeur de la surface 11 supérieure de la partie métaphysaire 2 ; elle est par ailleurs plus effilée du côté de l'intrados I de la prothèse que la surface 11. Ceci permet de caler la partie de col dans plusieurs orientations angulaires autour de l'axe de l'orifice traversant 17 sans que celle-ci déborde de la partie métaphysaire. Cependant dans une variante avec collerette 19 de la partie éphiphysaire, la longueur D de cette surface 11 prise depuis l'axe de l'orifice traversant jusqu'à son extrémité du côté de l'intrados de la prothèse peut être supérieure à cette même dimension d de la partie métaphysaire. On peut ainsi obtenir une sorte d'épaulement de la prothèse une fois assemblée qui vient comprimer la partie d'os subsistant à cet endroit. Cette compression est semble-t-il favorable à un meilleur ancrage de la prothèse.

## Revendications

1. Tige de reconstruction pour prothèse de hanche comportant un clou (1) longitudinal destiné à une implantation diaphysaire avec au moins un orifice transversal (6), une partie métaphysaire (2) adaptable à l'extrémité supérieure du clou (1) et une partie épiphysaire(3) surmontant la partie métaphysaire, l'ensemble étant assemblé par l'intermédiaire d'une vis (4) traversante commune, **caractérisée en ce que** la partie métaphysaire (2) présente une asymétrie par rapport à son plan (P) frontal médian passant par l'axe de l'orifice (10) de la vis traversante, afin de pouvoir correspondre au mieux avec la géométrie naturelle de fémur à reconstruire.

2. Tige selon la revendication 1, **caractérisée en ce que** la partie métaphysaire (2) comporte une surface plane supérieure (11) et un pion de centrage (12) de cette dernière autour de l'orifice de la vis traversante, en saillie de cette surface (11) et pourvu au moins en partie inférieure d'une pluralité de crans (14) d'indexation angulaire de la partie épiphysaire autour de ce pion.

3. Tige selon la revendication 2, **caractérisée en ce que** la partie épiphysaire (3) est limitée par une surface plane inférieure (16) dont la partie voisine de l'intrados de la prothèse, sous le col, est plus étroite que la partie correspondante de la surface plane supérieure (11) de la partie métaphysaire (2).

4. Tige selon l'une des revendications précédentes, **caractérisée en ce que** le clou (1) possède à son extrémité supérieure un moyen d'indexation (8) angulaire du matériel ancillaire de mise en place d'au moins une broche (5) dans l'orifice transversal (6) susdit.

## Patentansprüche

1. Wiederherstellungsschaft für eine Hüftprothese, umfassend einen in Längsrichtung verlaufenden Nagel (1), der zur diaphysären Implantation bestimmt ist und mindestens eine Queröffnung (6) hat, einen metaphysären Abschnitt (2), der an das obere Ende des Nagels (1) anpassbar ist, sowie einen epiphysären Abschnitt (3), der über dem metaphysären Abschnitt angeordnet ist, wobei der Satz mittels einer gemeinsamen Durchgangsschraube (4) zusammengesetzt wird, **dadurch gekennzeichnet, dass** der metaphysäre Abschnitt (2) in Bezug auf seine Front-Mittelebene (P), die durch die Achse der Öffnung (10) für die Durchgangsschraube verläuft, eine Asymmetrie aufweist, um bestens mit der natürlichen Geometrie des wiederherzustellenden Oberschenkelknochens übereinstimmen zu können.

2. Schaft nach Anspruch 1, **dadurch gekennzeichnet, dass** der metaphysäre Abschnitt (2) eine ebene, obere Fläche (11) und einen Zentrierzapfen (12) zum Zentrieren dieser Fläche um die Öffnung für die Durchgangsschraube umfasst, der von dieser Fläche (11) absteht und zumindest im unteren Teil mit einer Vielzahl von Kerben (14) zur Winkelindexierung des epiphysären Abschnittes um diesen Zapfen versehen ist.

3. Schaft nach Anspruch 2, **dadurch gekennzeichnet, dass** der epiphysäre Abschnitt (3) von einer ebenen unteren Fläche (16) begrenzt ist, deren Abschnitt, der unter dem Hals zur inneren Wölbung der Prothese benachbart ist, schmäler ist als der entsprechende Abschnitt der ebenen, oberen Fläche (11) des metaphysären Abschnittes (2).

4. Schaft nach einem der vorhergehenden Ansprüche, **dadurch gekennzeichnet, dass** der Nagel (1) an seinem oberen Ende Mittel (8) zur Winkelindexierung des Hilfsgerätes zum Einsetzen mindestens eines Stiftes (5) in die oben genannte Queröffnung (6) hat.

## Claims

1. A reconstruction rod for a hip prosthesis, the rod comprising a longitudinal shank (1) for diaphyseal implantation and having at least one transverse orifice (6), a metaphyseal portion (2) suitable for fitting to the top end of the shank (1), and an epiphyseal portion (3) surmounting the metaphyseal portion, the assembly being assembled together by means of a common through screw (4), the rod being **characterized in that** the metaphyseal portion (2) presents asymmetry relative to its front midplane (P) obtaining the axis of the orifice (10) for the through screw, so as to be capable of corresponding as well as possible to the natural shape of the femur that is to be reconstructed.

2. A rod according to claim 1, **characterized in that** the metaphyseal portion (2) comprises a plane top surface (11) and a peg (12) for centering the surface about the orifice for the through screw, the peg projecting from said surface (11) and being provided, at least in its bottom portion, with a plurality of index notches (14) for angularly indexing the epiphyseal portion about said peg.

3. A rod according to claim 2, **characterized in that** the epiphyseal portion (3) is defined by a plane bottom surface (16) whose portion adjacent to the curved undersurface of the prosthesis beneath the neck is narrower than the portion corresponding to the plane top surface (11) of the metaphyseal portion (2).

4. A rod according to any preceding claim, **characterized in that** the shank (1) presents indexing means (8) at its top end for angularly indexing auxiliary equipment for installing at least one pin (5) in the above-mentioned transverse orifice (6).
